# EUROPEAN PATENT SPECIFICATION

(11) **EP 2 229 445 B1**
(45) Date of publication and mention of the grant of the patent: **05.12.2012**
(21) Application number: 08860093.7
(22) Date of filing: 12.12.2008
(51) Int. Cl.: C12N 15/11, C12N 15/87

(54) **MEANS FOR DELIVERY OF NUCLEIC ACIDS ACTIVE FOR GENE SILENCING USING SYNTHETIC POLYMERS**
MITTEL ZUR ZUFÜHRUNG VON FÜR GEN-SILENCING AKTIVEN NUKLEINSÄUREN UNTER VERWENDUNG SYNTHETISCHER POLYMERE
MOYEN DE DIFFUSION D'ACIDES NUCLEIQUES ACTIFS DANS LE SILENÇAGE GENIQUE AU MOYEN DE POLYMERES SYNTHETIQUES

(30) Priority: 13.12.2007 US 13358 P
(43) Date of publication of application: 22.09.2010
(73) Proprietor: POLYPLUS TRANSFECTION, 67401 Illkirch (FR)
(72) Inventor: ADIB, Abdennaji, F-67401 Illkirch (FR); ERBACHER, Patrick, F-67230 Benfeld (FR); STOCK, Fabrice, F-67230 Benfeld (FR); HAFDI, Nadia, F-67401 Illkirch (FR)
(74) Representative: Paris, Fabienne
(86) International application number: PCT/IB2008/055256
(87) International publication number: WO 2009/074970

(56) References cited:
- WO-A1-2005/116045
- WO-A2-2008/007073
- READ MARTIN L ET AL: "A versatile reducible polycation-based system for efficient delivery of a broad range of nucleic acids" NUCLEIC ACIDS RESEARCH, vol. 33, no. 9, 2005, XP002525419 ISSN: 0305-1048
- BETTINGER T ET AL: "Peptide-mediated RNA delivery: a novel approach for enhanced transfection of primary and post-mitotic cells" NUCLEIC ACIDS RESEARCH, OXFORD UNIVERSITY PRESS, SURREY, GB, vol. 29, no. 18, 15 September 2001 (2001-09-15), pages 3882-3891, XP002390292 ISSN: 0305-1048
- DATABASE EPODOC EUROPEAN PATENT OFFICE, THE HAGUE, NL; XP002539578 & WO 2007/029361 A1 (TOKYO UNIVERSITY OF PHARMACY A [JP]; OKADA HIROAKI [JP]; TAKASHIMA YUK) 15 March 2007 (2007-03-15)
- DATABASE MEDLINE [Online] US NATIONAL LIBRARY OF MEDICINE (NLM), BETHESDA, MD, US; 2008, CREUSAT GAELLE ET AL: "Tyrosine-modified PEI: a novel and highly efficient vector for siRNA delivery in mammalian cells." XP002525422 Database accession no. NLM18776268 & NUCLEIC ACIDS SYMPOSIUM SERIES (2004) 2008, no. 52, 2008, pages 91-92, ISSN: 1746-8272
- CREUSAT GAELLE ET AL: "Self-Assembling Polyethylenimine Derivatives Mediate Efficient siRNA Delivery in Mammalian Cells" CHEMBIOCHEM, vol. 9, no. 17, November 2008 (2008-11), pages 2787-2789, XP002525420 ISSN: 1439-4227
- AMY C RICHARDS GRAYSON ET AL: "Biophysical and Structural Characterization of Polyethylenimine-Mediated siRNA Delivery in Vitro" PHARMACEUTICAL RESEARCH, vol. 23, no. 8, 15 July 2006 (2006-07-15), pages 1868-1876, XP019405163 KLUWER ACADEMIC PUBLISHERS-PLENUM PUBLISHERS, NE ISSN: 1573-904X
- DATABASE MEDLINE [Online] US NATIONAL LIBRARY OF MEDICINE (NLM), BETHESDA, MD, US; May 1999 (1999-05), ERBACHER P ET AL: "Transfection and physical properties of various saccharide, poly(ethylene glycol), and antibody-derivatized polyethylenimines (PEI)." XP002525423 Database accession no. NLM10738569 & THE JOURNAL OF GENE MEDICINE 1999 MAY-JUN, vol. 1, no. 3, May 1999 (1999-05), pages 210-222, ISSN: 1099-498X
- LI SHUFENG ET AL: "Polyethylenimine-complexed plasmid particles targeting focal adhesion kinase function as melanoma tumor therapeutics." MOLECULAR THERAPY : THE JOURNAL OF THE AMERICAN SOCIETY OF GENE THERAPY, vol. 15, no. 3, March 2007 (2007-03), pages 515-523, XP002525421 ISSN: 1525-0024

### SEQUENCE LISTING

<110> POLYPLUS-TRANSFECTION
<120> MEANS FOR DELIVERY OF NUCLEIC ACIDS ACTIVE FOR GENE SILENCING USING SYNTHETIC POLYMERS
<130> 62716
<150> US 61/013 358
   <151> 2007-12-13
<160> 8
<170> PatentIn version 3.3
<210> 1
   <211> 23
   <212> DNA
   <213> Artificial sequences
<220>
   <223> Oligonucleotides
<220>
   <221> misc_feature
   <222> (20)..(22)
   <223> deoxyribonucleotides
<400> 1
   cuuacgcuga guacuucgad tdt 23
<210> 2
   <211> 23
   <212> DNA
   <213> Artificial sequences
<220>
   <223> Oligonucleotides
<220>
   <221> misc_feature
   <222> (1)..(3)
   <223> deoxyribonucleotides
<400> 2
   dtdtgaaugc gacucaugaa gcu 23
<210> 3
   <211> 23
   <212> DNA
   <213> Artificial sequences
<220>
   <223> oligonucleotides
<220>
   <221> misc_feature
   <222> (20)..(22)
   <223> deoxyribonucelotides
<400> 3
   cguacgcgga auacuucgad tdt 23
<210> 4
   <211> 23
   <212> DNA
   <213> Artificial sequences
<220>
   <223> oligonucleotides
<220>
   <221> misc_feature
   <222> (1)..(3)
   <223> deoxyribonucleotides
<400> 4
   dtdtgcaugc gccuuaugaa gcu 23
<210> 5
   <211> 23
   <212> DNA
   <213> Artificial Sequences
<220>
   <223> oligonucleotides
<220>
   <221> misc_feature
   <222> (20)..(22)
   <223> deoxyribonucleotides
<400> 5
   gcaccacuag uugguugucd tdt 23
<210> 6
   <211> 23
   <212> DNA
   <213> Artificial sequences
<220>
   <223> Oligonucleotides
<400> 6
   dtdtcguggu gaucaaccaa cag 23
<210> 7
   <211> 23
   <212> DNA
   <213> Artificial sequences
<220>
   <223> oligonucleotides
<220>
   <221> misc_feature
   <222> (20)..(22)
   <223> deoxyribonucleotides
<400> 7
   cuggacuucc agaagaacad tdt 23
<210> 8
   <211> 23
   <212> DNA
   <213> Artificial sequences
<220>
   <223> Oligonucleotides
<220>
   <221> misc_feature
   <222> (1)..(3)
   <223> deoxyribonucleotides
<400> 8
   dtdtgaccug aaggucuucu ugu 23

## Description

The invention relates to compositions for efficient synthetic polymer-mediated delivery to eukaryotic cells in culture ,*in vivo* or *ex vivo* of nucleic acids mediating gene silencing in cells, particularly small interfering RNA (designated as siRNA in the following description and the claims) providing RNA interference (RNAi)and optionally plasmid DNA.

RNA interference (RNAi) is a technology for gene silencing at the early gene function level, the mRNA (Fire et al, 1998). The principle is an extremely selective interaction of short RNA duplexes (siRNA; small interfering RNA) with a single target in the mRNA, providing sequence-specific mRNA degradation and thus inhibition of protein production.

RNAi is highly effective due to a predictable design of active sequences of siRNA and to the targeting of mRNA. When siRNA duplexes are introduced by transfection with a vector, transfection reagent, and delivered into the cytoplasm, RNAi has been shown to effectively silence both exogenous and endogenous genes in a variety of mammalian cells, including cell lines (Elbashir et al, 2001) as well as primary cells.

RNAi is a powerful tool for human therapy which would dramatically drop developments of new therapy approaches for severe diseases such as cancer or viral infections. For exploiting the vast potential of RNAi, the generation of RNAi transfection vectors and strategies developed for efficient delivery to cells and tissues of diseased organisms is required.

The success of RNAi depends on both siRNA (design and chemistry) and vector/carrier for cell delivery. As compared to antisense or ribozyme technology, the secondary structure of the target mRNA (may not be) is not a strong limiting factor for silencing with siRNA. Many sequences of siRNA may be effective for one targeted mRNA. The stability of siRNA duplexes and the amount of siRNA delivered to cells is the most limiting factors for silencing rather the target accessibility by the chosen sequence. Two approaches are proposed for introducing siRNA into cells: the delivery by transfection of synthetic siRNA duplexes into the cytoplasm of cells and the delivery of siRNAs expressed *in situ* from a plasmid (or DNA cassettes) preliminary introduced by gene transfer into the nucleus.

RNAi in mammalian cells depends upon efficient intracellular delivery of either siRNAs or DNA vector expressing si/shRNAs or microRNA-adapted short hairpin RNA (shRNAmir)(Sui et al., 2002 ; Yu et al., 2002 ; Miyagishi & Taira, 2002 ; Silva et al., 2005 ; Brummelkamp et al., 2002). Expression of shRNAs (short hairpin RNAs) or siRNAs in mammalian cells can be achieved via transcription from either Pol II or Pol III (U6 or H1) promoters. DNA vectors are based on plasmid and viral vector systems that express double-stranded short hairpin RNAs (shRNAs) that are subsequently processed to siRNAs by the cellular machinery. Recent developments of shRNA systems allow tissue-specific and inducible knockdown of genes. Intracellular delivery of such DNA vectors expressing active RNAs for RNA interference can be achieved by using recombinant viruses or non-viral delivery systems.

Coming to the gene delivery technology, potent viral or non-viral vectors are useful for introducing siRNA duplexes in cells. For mammalian cells in culture, viral vectors appear a potent tool for the production of an intracellular pool of siRNAs expressed from delivered plasmid DNA because of their transduction efficiency and facility to deliver DNA into the nucleus. However, recombinant viral delivery systems still suffer from their immunogenicity and potential risk in clinical situations. In contrast, the transfection of nucleic acids (plasmids or synthetic siRNAs) with synthetic systems is a versatile method showing flexibility and absence of immunogenicity. The transfection of synthetic siRNA duplexes (chemically or enzymatically produced) with non-viral vectors is the best technology to delivery mainly short double-stranded RNA into the cytoplasm. The most efficient non-viral vectors for siRNA delivery are based on cationic lipids-mediated transfection coming initially from the field of gene delivery or newly developed for the specific RNAi application. Cationic lipids formulations compact nucleic acids (plasmid, oligonucleotides, siRNA duplexes) into positively charged particles capable of interacting with anionic proteoglycans at the cell surface and entering cells by endocytosis. After trafficking towards intracellular vesicles of endocytosis, mainly endosomes, the cationic lipids have the property to destabilize the membrane of these intracellular compartments by lipids exchange/diffusion allowing a nucleic acids 'decomplexation' and release into cytoplasm (Xu and Szoka, 1996). In addition, a secondary mechanism called proton sponge activity may be associated with some lipids inducing endosomes swelling and rupture which release thus nucleic acids in the cytoplasm. As RNAi mechanism occurs in the cytoplasm, vectors based on formulation of cationic lipids are efficient vehicles to deliver synthetic siRNA duplexes into cells. For siRNA expressed *in situ* from plasmid, non-viral vectors, based on cationic lipids formulations or cationic polymers, destabilizing endosomal compartments are suitable.

In contrast to their ability to transfect efficiently a gene (long double stranded DNA) into cells, cationic polymers are poorly efficient for the delivery of short nucleic acid. Cationic polymers are shown to be less efficient for siRNA delivery than cationic lipid-based systems. Cationic polymers are able to mediate RNA interference *in vitro* with concentrations of siRNA around 100 to 200 nM. Selectivity of RNA interference at such concentrations is a limit of their use. In addition, the high amount of siRNA used is correlated with a high amount of polymer which induces cytotoxic effects. To date, cationic polymers such as branched or linear polyethylenimines, poly-histidyl polymers, chitosan, poly(amino ester glycol urethane), amino cyclodextrin derivatives were used *in vitro* but without relevant efficiency compared to cationic lipids.

WO2005116045 discloses the delivery of siRNA molecules with a polycation (exemplifed with inter alia, a polyethyleneimine), in combination with an 'amphipathic compound'. Addition of latter said to minimise cellular toxicity and enhance efficiency of siRNA delivery.

An objective of the inventors was to increase the potency of cationic polymers, (specifically polyethyleneimines) this major class of non-viral delivery vectors, for *in vitro* siRNA transfection. Cationic polymers are able to interact via electrostatic interactions between the phosphates of siRNA and the amino groups of polymer. However, according to the structure of siRNA, polymers are unable to condense such small double helix comprising only two turns (about 20 nucleotides per strand). Even complexation occurs between siRNA and cationic polymer leading to siRNA sticking along the polyamine backbone, cationic polymers lack cooperative interactions to induce a condensation into particles or micro-aggregates of molecules complexed.

In addition to electrostatic interactions between positive and negative charges bearing by the polyamine and the small double stranded oligonucleotide, hydrophobic stacking with the nucleic bases and hydrogen bond forming interactions is a way we propose to increase interactions between polyamine and siRNA. Taking together, electrostatic and hydrophobic interactions as well as hydrogen bonds provide enough energy leading to stable complexation and condensation of siRNAs.

Aromatic amino acids (AAA) are responsible of the hydrophobic characteristics in protein and are involved in interactions between protein-protein and protein-ligand via hydrophobic interactions. AAAs are also able to interact with nucleic acid by stacking with the nucleic bases (guanidine, adenosine, thymine or cytosine).

The invention relates to a new concept of hydrophobic polyamines which comprised a polyethyleneimine (branched or linear) backbone highly modified with aromatic amino acids. This kind of polymer offers the possibility to interact with small nucleic acids, like siRNAs, via electrostatic interactions, hydrophobic stackings and hydrogen bonds. As a barrier of energy to overcome, addition by chemical grafting of AAA to polyethyleneimine will be able to provide the sufficient energy to induce cooperative interactions ending in condensation. Consequence is the stabilization of the complex generated by hydrophobic interaction under stable particles or aggregates.

The present invention describes a new class of non viral transfection agents, belonging to the polyethyleneimine group, which are particularly adapted for the transfection of small sized oligonucleotides. Especially the physical properties of small oligonucleotides prompted the inventors to design a new class of transfection agents based on hydrophobic and cationic polymers.

The inventors have found that transfection agents of high efficiency could be obtained by combining an oligonucleotide of interest with hydrophobic and cationic polyethyleneimines forming stable complexes of transfection.

Advantageously, said agents are also useful for co-transfection of siRNA with plasmid DNA that can promote in situ expression of small RNAs mediating RNA interference.

It is then an object of the invention to provide new compositions useful as transfection agents for siRNA and optionally DNA vector expressing active RNAs for RNAi.

The invention also relates to a method of transfection of cells *in vitro.*

The compositions of the invention useful as transfection agents comprise polyethyleneimines (branched or linear) modified by aromatic amino acids and small double-strand or single-strand RNA.

Preferably, the molecular weight of said polyethyleneimines is above 400 Da.

Useful polyethyleneimines comprise linear polyethylenimine of 2 KD to 220 KD.

The aromatic amino acids used to modify the polyamines are selected in the group comprising tyrosine, tryptophan and phenylalanine or the derivatives thereof.

Preferably, the aromatic amino acids are tryptophan and/or tyrosine.

In the above defined compositions, the RNA is normal or modified, the modification groups being for example 2'-Fluo, 2'-Methoxy, phosphorothioate, LNA or morpholino.

The above defined RNA is double stranded or single stranded antisense siRNA or mixtures of single stranded sense antisense siRNA.

Advantageously, the siRNA has 15-30 mers.

A preferred composition comprises the polyethyleneimines modified by aromatic amino acids such as above defined and double stranded or single stranded siRNA in an isotonic medium, for example NaCl, glucose, a buffer.

The concentration of siRNA may vary from picomolar to micromolar.

Advantageously, the above defined compositions comprise one or several additives such as PEG, PVA, saccharide, polysaccharide, peptide, protein, vitamins.

The siRNA can comprise groups stabilized against degradation with suitable groups, selected in the group comprising purine nucleotides, pyrimidine nucleotides substituted by modified analogs such as deoxynucleotides, and/or modified nucleotide analogs such as sugar- or backbone modified ribonucleotides or deoxyribonucleotides. The oligonucleotides sequences can contain deoxyribonucleotides, ribonucleotides or nucleotide analogs (Verma and Eckstein, 1998), such as methylphosphonate, morpholino phosphorodiamidate, phosphorothioate, PNA, LNA, 2'alkyl nucleotide analogs.

It is another object to provide a process for the synthesis of said compositions.

The method for synthesizing the polyethyleneimines modified by aromatic amino acids of the above defined compositions comprise the use of super-ester of aromatic amino acids activated by Dimethoxytriazine-N-methylmorpholium (DMTMM) in the presence of the polyamines.

Advantageously, synthesis of the polyethyleneimines is carried out in a basic buffer such as a borate buffer 200 mM, pH= 7.5-9 or in an aqueous medium in the presence of a base or a water/alcohol mixture.

The percentage of modification of polyethyleneimines by aromatic amino acids in said composition more particularly varies from 0.01% to 100%, particularly of 15% to 50%.

The invention also relates to a method for *in vitro* or *ex-vivo* transferring siRNA or siRNA and plasmid DNA, comprising using a composition such as above defined.

The *in vitro* transfer of siRNA is advantageously carried out in a medium culture containing adherent cells or cells in suspension.

The medium is a normal medium or synthetic medium.

The invention also provides compositions for use as pharmaceutical compositions for inducing a regulating effect on the expression of one or more target proteins responsible or involved in genetic hereditary diseases or complex genetic diseases.

Other characteristics and advantages of the invention are given in certain of the following examples wherein it is referred to Figures 1-8, which respectively relate to:
- Figure 1: ¹H-NMR analysis of L-PEI-Tyr conjugate in D₂O.
- Figures 2A and 2B: siRNA delivery in A549 cells.
- Figure 3: RNA interference efficiency of luciferase gene (pGL3) stably expressed by A549-GL3Luc cells by GL3Luc siRNA transfected with the L-PEI_{10K} modified with different extents of tyrosine residue.
- Figure 4: comparative silencing efficiency of luciferase gene (pGL3) stably expressed by A549-GL3Luc cells by GL3Luc siRNA transfected with the L-PEI_{10K} or L-PEI_{10K}Tyr_{33%} conjugate.
- Figure 5: Selective RNA interference of luciferase gene (pGL3) stably expressed by A549-GL3Luc cells by GL3Luc siRNA transfected with the L-PEI_{10K}-Tyr_{33%}.
- Figure 6: Efficient and selective GAPDH gene silencing in HeLa cells lines after transfection of siRNA complexed with 1-PEI_{10K}-Tyr_{33%}.
- Figure 7: Selective RNA interference of luciferase gene (pGL3) stably expressed by A549-GL3Luc cells by GL3Luc siRNA transfected with the PAA_{17K}-Tyr_{40%}.
- Figures 8A and 8B: Selective RNA interference of luciferase gene (GL2Luc) expressed by HeLa cells after co-transfection of GL2Luc siRNA and pCMVLuc plasmid (pGL2Luc) with the PEI_{10K}-Tyr_{19%}.

Those examples pertaining to modified polyethyleneimines are illustrative of the invention. Other examples are provided for information purposes only.

### Material and Methods

### Chemicals and oligonucleotides

Oligonucleotides were chemically synthesised and PAGE purified by Eurogentec (Belgium). Oligonucleotides were annealed in 1x Annealing buffer (50 mM K-Acetate, 50 mM Mg-Acetate) (Eurogentec) for 2 min. at 95°C, followed by 2-4 hours incubation at room temperature. GAPDH SMART pool® reagent was from Dharmacon.

SiRNA duplexes used correspond to sequences SEQ ID N°1 and SEQ ID N°2; SEQ ID N°3 and SEQ ID N°4; SEQ ID N°5 and SEQ N°6; SEQ ID N°7 and SEQ ID N°8

| | |
|---|---|
| GL3Luc siRNA duplex | SEQ ID N°1 5'-CUUACGCUGAGUACUUCGA(dT)₂-3' |
| | SEQ ID N°23'-(dT)₂GAAUGCGACUCAUGAAGCU-5' |
| GL2Luc siRNA duplex | SEQ ID N°3 5'-CGUACGCGGAAUACUUCGA(dT)₂-3' |
| | SEQ ID N°4 3'-(dT)₂GCAUGCGCCUUAUGAAGCU-5' |
| siRNA TNF-alpha Rhodamine duplex | SEQ ID N°5 5'- GCACCACUAGUUGGUUGUC(dT)₂-3' |
| | SEQ ID N°6 3'- dT)₂CGUGGUGAUCAACCAACAG-5' |
| Lamin A/C siRNA duplex | SEQ ID N°75'-CUGGACUUCCAGAAGAACAdTdT-3' |
| | SEQ ID N°83'-dTdTGACCUGAAGGUCUUCUUGU-5' |

All reagents for chemistry and starting material were purchased from Sigma-Aldrich (France) and were used without prior purification. Solvents were ordered from SOS-Carlo Erba (France). Diethylether was dried and distilled over sodium benzophenone.

¹H NMR spectra were recorded with a Bruker AF-400 spectrometer at 25 °C in CDCl₃, D₂O or CD₃OD and proton chemical shits are reported downfield from TMS. NMR multiplicities are abbreviated as s = singlet, d= doublet, br= broad, m = multiplet, t = triplet

### Synthesis of linear polyethylenimine (L-PEI)

LPEI is obtained from the intermediate poly-2-ethyl-2-oxazoline generated after the living cationic ring opening polymerization of 2-ethyl-2-oxazoline monomer.

*Synthesis of poly-2-ethyl-2-oxazoline:* 0.4 moles 2-ethyl-2-oxazoline monomer were dissolved in 40 ml acetonitrile then 0.4/X moles of methyl p-toluenesulfonate are added under argon atmosphere. The reaction was heated at 80°C for 24-48 hours. The reaction was quenched with saturated acqueous Na₂CO₃ and heated for 24 hours. After slow cooling at room temperature, 10 ml of methanol and ether were added until precipitation. The precipitate was filtered and washed with ether. Poly-2-ethyl-2-oxazoline was obtained with 80-90% yield. ¹H-NMR analysis, 400 MHz, in CDCl₃: 1-1.06 ppm (s, 3H, **CH₃**CH₂CONCH₂CH₂) ; 2.2-2.3 ppm (m, 2H, CH₃**CH₂**CONCH₂CH₂) ; 3-3.4 ppm (s, 4H, CH₃CH₂CON**CH₂CH₂**).

Synthesis of L-PEI: 0.35 moles of poly-2-ethyl-2-oxazoline were dissolved in 100 mL of water, then 200 mL of hydrochloric acid 37% were added and the mixture is heated at 120°C. After 24 hours, the reaction mixture was evaporated and then water was added before lyophilisation. The yield was 90%. Analysis by ¹H-NMR, 400 MHz in D₂O: single peak at 3.4 ppm

| 0.4/X moles of methyl *p*-toluenesulfonate | X= number of monomer | Mw of poly-2-ethyl-2-oxazoline | Mw of polyethylenimine |
|---|---|---|---|
| 8 mmoles | 50 | 5 KDa | 2 KDa |
| 1.6 mmoles | 250 | 25 KDa | 10 KDa |
| 0.8 mmoles | 500 | 47 KDa | 20 KDa |

### Synthesis of polyethylenimine-aromatic α-amino acid conjugate

### Synthesis of N,O-Boc-tyrosine

Five g of L-tyrosine (Sigma) are dissolved in 125 mL of Na₂CO₅ (0.1 mg/mL) and 50 mL of THF. Then, 17.2 g of BoC₂O dissolved in 75 mL of THF are added onto the tyrosine solution and the mixture was stirred for 3 days at room temperature. Water (40 ml) was added and N,O-Boc-tyrosine was extracted with ether. The aqueous phase was acidified with HCl and 2 novel extractions with ethyl acetate were performed. After evaporation, the raw product is purified by chromatography on silica gel (in 5% MeOH/CH₂Cl₂). 5.2 g of N,O-BOC-Tyrosine were obtained.

Analysis by ¹H-NMR, 400 MHz, in CDCl₃: 7.2 ppm (d, 2H, aromatic H), 7.1 ppm (d, 2H, aromatic H), 4 ppm (m, 1H, BocO-Ar-CH₂-C**H** (NHBOC) -COOH), 3-2.99 ppm (dd, 1H, BocO-Ar-C**H₂**-CH(NHBOC)-COOH), 2.84-2.78 ppm (dd, 1H, BocO-Ar-C**H₂**-CH(NHBOC)-COOH), 1.48 ppm (S, 9H, **Boc**O-Ar-CH₂-CH(NHBOC), 1.32 ppm (S, 9H, BocO-Ar-CH₂-CH(NH**BOC**)-COOH).

### Synthesis of N-Boc-tryptophane

Synthesis of N-Boc-tryptophane was realized with the same protocol used for the synthesis of N,O-Boc-tyrosine, starting with 2g of tryptophane and 6.5g of BoC₂O, to give 2.79g of white solid

Analysis by ¹H-NMR, 400 MHz, in CDCl₃: 8.1 ppm (s, 1H, - COO**H**) 7.6 ppm (d, H, aromatic H), 7.38 ppm (d, 1H, aromatic H), 7.2 ppm (m, 1H, aromatic H), 7.16 (m, 1H, aromatic H), 7.01 ppm (m, 1H, aromatic H), 5.09 ppm (d, 1H, N**H**BOC), 4.68 ppm (br, 1H, BocO-Ar-CH₂-C**H**(NHBOC)-COOH), 3.35 ppm (m br, 2H, BocO-Ar-C**H₂**-CH(NHBOC)-COOH), 1.45 ppm (S, 9H, **Boc**O-Ar-CH₂-CH(NHBOC).

### Synthesis of polyethylenimine-tyrosine conjugate (L-PEI-Tyr)

### Synthesis of L-PEI_{10KD}-Tyr_{33%}

**Protocol 1**: 100 mg of L-PEI_{10K}.HCl (1.26 mmoles) were dissolved in 5 mL of 200 mM borate buffer, pH=8.2. Then, pH was adjusted to 8 with 10N NaOH. 240 mg of N,O-Boc-Tyrosine (0.632 mmoles) dissolved in 15 mL of THF were added and the mixture was stirred for 10 minutes. DMTMM (500 mg) was added into the mixture and stirred for 48 hours. After evaporation, the solid was washed with water. After drying, 239 mg of yellow solid were obtained (L-PEI_{10K}-TyrBoc₂).
185 mg of LPEI-TyrBoc₂ were dissolved in 5 mL of trifluoroacetic (TFA). After 3 hours, the reaction mixture was evaporated and dialysed in water.
After lyophilization, 111 mg of white solid was obtained (L-PEI_{10K}-Tyr)

**Protocol 2**: To 200 mg de L-PEI_{10K}.HCl (2.53 mmoles) in 4 mL of water, 0.56 mL of N-methylmorpholine and 483 mg de N,O-Boc-Tyrosine (0.5 equivalent, 1.26 mmoles) in 12 mL of methanol were added. The reaction mixture were stirred for 30 minutes and 700 mg of DMTMM were added. After 48 hours, the reaction mixture was evaporated and the solid was dissolved in 8 mL of TFA. After 3 hours, the reaction mixture was evaporated and then dialysed in water one day and in HCl 2N two days. After lyophilization, 260 mg of white solid was obtained.

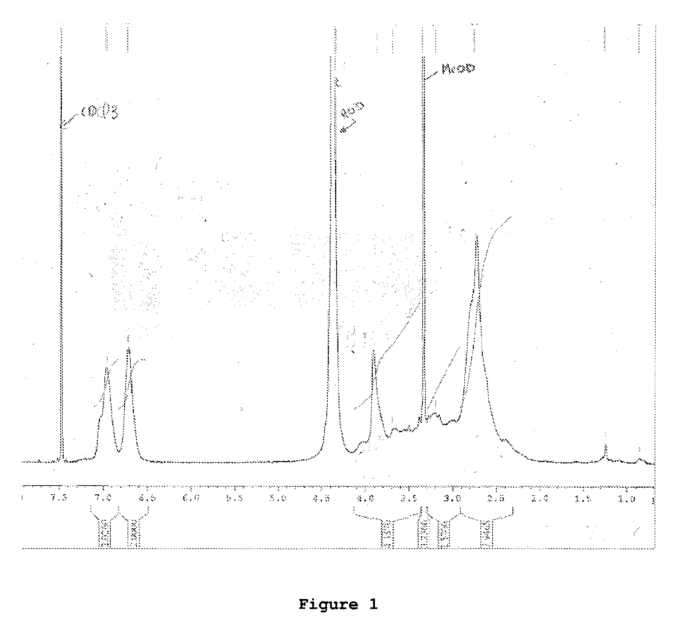

¹H NMR, 400 MHz, CDCl₃ (Figure 1): 6.9 ppm (s br, 2H, H aromatic), 6.7 ppm (s br, 2H, H aromatic), 3.8 ppm (s, 4H, TyrCONC**H₂CH₂**), 3.68 ppm (br, 1H, ArCH₂C**H**(NH₂)COPEI), 3.1 ppm (br, 2H, ArC**H₂**CH(NH₂) COPEI), 2.74 ppm (s br, 4H, NH**CH₂CH₂**).

### Synthesis of polyethylenimine-tryptophan conjugate (L-PEI-Trp)

### Synthesis of L-PEI_{10KD}-Trp_{33%}

Synthesis of L-PEI-Trp was realized with the same protocol used for the synthesis of L-PEI-Tyr, starting with 0.1 g of LPEI and 0.193 g of N-Boc-tryptophane.
¹H NMR, 400 MHz, D₂OD : 7.4-7.1 ppm (m br, 5H, H aromatic), 3. 91 ppm (m br, 3H, ArC**H₂**C**H**(NH₂)COPEI), 3.47 ppm (s br, 4H, ArCON**CH₂CH₂** and HN**CH₂CH₂**).

### Cell culture

HeLa (human cervix epithelial adenocarcinoma, CCl-2) cells were grown in MEM (Eurobio) supplemented with 2 mM glutamax (Eurobio), Earle's BSS (Eurobio), 1.5 g/L sodium bicarbonate (Eurobio), 0.1 mM non-essential amino acids (Eurobio), 1.0 mM sodium pyruvate (Eurobio), 100 units/ml penicillin (Eurobio), 100 µg/ml streptomycin (Eurobio), and 10% of FBS (Perbio).

A549 (human lung carcinoma, ATCC N° CCL-185) cells stably expressing the GL3 luciferase (*Photinus pyralis* luciferase under the control of SV40 elements) were obtained after stable transfection of pGL3Luc plasmid (Clontech). A549-GL3Luc cells were grown in RPMI-1640 and supplemented with 10% fetal bovine serum, 2 mM glutamax, 100 units/ml penicillin, 100 µg/ml streptomycin and 0.8 µg/ml G418 (Promega). All the cells were maintained at 37°C in a 5% CO₂ humidified atmosphere.

### Transfection experiments

One day before transfection, 2.5 x 10⁴ cells were seeded in 24-well tissue culture plate in 1 ml fresh complete medium containing 10% FBS. Before transfection, complexes of siRNA/polymer were prepared. The desired amount of siRNAs was diluted in 50 µl of 50 mM phosphate buffer, pH 6 or 8. Then, the desired volume of polymer solution (7.5 mM nitrogen) was added into the siRNA solution. The resulting solution was mixed with a Vortex for 10 seconds and left for 10-15 minutes at room temperature. Before adding the transfection solution onto the cells, the complete medium was removed and replaced by 0.55 ml of fresh complete medium containing 10 % FBS. Then, 50 µl of complexes solution were added per well and the plates were incubated at 37°C.

### Co-transfection experiments

One day before transfection, 5 x 10⁴ cells were seeded in 24-well tissue culture plate in 1 ml fresh complete medium containing 10% FBS. Before transfection, complexes with polymer, plasmid and siRNA were prepared. One hundred ng of pCMVLuc (GL2Luc duplex sequence) desired amount of siRNAs were diluted in 50 µl of 50 mM phosphate buffer, pH 7. Then, 2 µl of 1-PEI_{10K}-Tyr_{19%} solution (7.5 mM nitrogen) were added into the plasmid and siRNA solution. The resulting solution was mixed with a Vortex for 10 seconds and left for 10-15 minutes at room temperature. Before adding the transfection solution onto the cells, the complete medium was removed and replaced by 0.55 ml of fresh complete medium containing 10 % FBS. Then, 50 µl of complexes solution were added per well and the plates were incubated at 37°C. Luciferase gene expression was measured after 24 h incubation period. Experiments were made in triplicates and the luciferase activity was expressed as Relative Light Unit (RLU) normalized by the content of protein in the cell lysates (mg of protein). Then, the silencing efficiency was calculated from the ratio of luciferase activities from GL2Luc siRNA- and GL3Luc siRNA-transfected cells.

### DNA vector based RNAi technology transfection protocol

One day before transfection, 5 x 10⁴ cells were seeded in 24-well tissue culture plate in 1 ml fresh complete medium containing 10% FBS. Before transfection, complexes with polymer and DNA vector expressing short RNA mediating RNAi (siRNA, shRNA, microRNA-adapted short hairpin RNA), were prepared. Plasmid RNAi vector (1 µg) was diluted in 50 µl of 50 mM phosphate buffer, pH 7. Then, 2-4 µl of l-PEI_{10K}-Tyr_{19%} solution (7.5 mM nitrogen) were added into the Plasmid RNAi vector solution. The resulting solution was mixed with a Vortex for 10 seconds and left for 10-15 minutes at room temperature. Before adding the transfection solution onto the cells, the complete medium was removed and replaced by 0.55 ml of fresh complete medium containing 10 % FBS. Then, 1 to 50 µl of complexes solution were added per well and the plates were incubated at 37°C. After one day of incubation, 0.4 ml of complete fresh medium was added. The level of the targeted gene expression (mRNA level) or inhibition of the protein production (protein level) was determined 24 to many days later. As a control, plasmid RNAi vector expressing a nonspecific active RNA (containing a mismatch sequence) against the targeted gene expression was used.

### Luciferase and protein assay

Luciferase gene expression was measured using a commercial kit (Promega, France). After removing the complete medium, three washings with 1 ml of PBS solution were made. Then, 100 µl of 1x lysis buffer were added per well, and the plate was incubated at room temperature for 30 minutes. The lysates were collected and centrifuged at 14,000 g for 5 minutes. The luciferase assay was assessed with 2.5 µl of lysate after injection of 100 µl of luciferin solution. The luminescence (RLU) was monitored with an integration over 5 seconds with a luminometer (LB960, Berthold, France). Results are expressed as light units integrated over 10 seconds (RLU), per mg of cell protein using the BCA assay (Pierce, France).

### Measurement of mRNA level

Messager RNA level was determined by the QuantiGene® Branched DNA assay (GenoSpectra) which is performed with whole cell lysates and without target amplification.

After 48h transfection, HeLa cells were washed with 1 mL PBS 1x (Cambrex) and lysed in 0.6 mL of 1x Genospectra lysis buffer for 30 min. at 50°C. Then, the plate was stored at -80°C for at least 30 min. The lysates were thawed and 2 to 20 µl of lysate were adding to the capture plate. Ten µl of lysis working reagent (for 48 reactions, the lysis working reagent is prepared by adding 25 µl of CE (capture extender), 25 µl of LE (label extender) and 25 µl of BL (blocking probe) and 425 µl of 3x lysis mixture, all compounds are from Genospectra) were added to the plate and the volume was completed to 100 µl with 1x lysis mixture. The plate was covered with a lid and incubated for 16h at 50°C. The plate was washed 3 times with 300 µl of 1x wash buffer (Genospectra), and 100 µl of Amplifier working solution (0.116 µl of amplifier diluted in 116 µl Amplifier diluent, all from Genospectra) were added to each well. The plate was incubated for 1 hour at 50°C. After 3 times 1x wash buffer washing, 100 µl of Label Probe Working Reagent (0.116 µl of label probe diluted in 116 µl Amplifier diluent, all from Genospectra) were added to each well and incubated for 1 hour at 50°C. The plate was then washed 3 times with 1x wash buffer and 100 µl of Substrate Working Reagent (0.348 µl of 10% Lithium Lauryl sulphate in 116 µl of Substrate, all from Genospectra) was added to each well. After 30 minutes incubation, the luminescence was measured in each well with a spectrophotometer (Berthold).

### Fluorescence microscopy

One day before transfection, 2.5 x 10⁴ A549 cells were seeded in 24-well tissue culture plate in 1 ml fresh complete medium containing 10% FBS. Before transfection, complexes of siRNA/polymer were prepared. The desired amount of siRNA-Rhodamine (Rho) was diluted in 50 µl of 50 mM phosphate buffer, pH 6 or 8. Then, the desired volume of L-PEI_{10K}-Tyr_{33%} solution (7.5 mM nitrogen) was added into the siRNA solution. The resulting solution was mixed with a Vortex for 10 seconds and left for 10-15 minutes at room temperature. Before adding the transfection solution onto the cells, the complete medium was removed and replaced by 0.55 ml of fresh complete medium containing 10 % FBS. Then, 50 µl of complexes solution were added per well and the plates were incubated at 37°C for 24 or 48 hours.

Before observation, cells were washed with 1ml of PBS-BSA1% and then observed by fluorescence microscopy (ECLIPSE TE2000-S, Nikon).

### Results

Linear polyethylenimine (L-PEI) having a mean molecular weight of 10 kDa was produced using cationic ring opening polymerization of 2-ethyl-2-oxazoline monomer. Then, L-PEI_{10K} was modified with tyrosine residues at various extents following the protocols 1 or 2 as described in the Material and Methods. All L-PEI-Tyr derivatives were characterized by ¹H-NMR as exemplified in Figure 1.

### SiRNA delivery into cells in culture with L-PEI-Tyr derivatives.

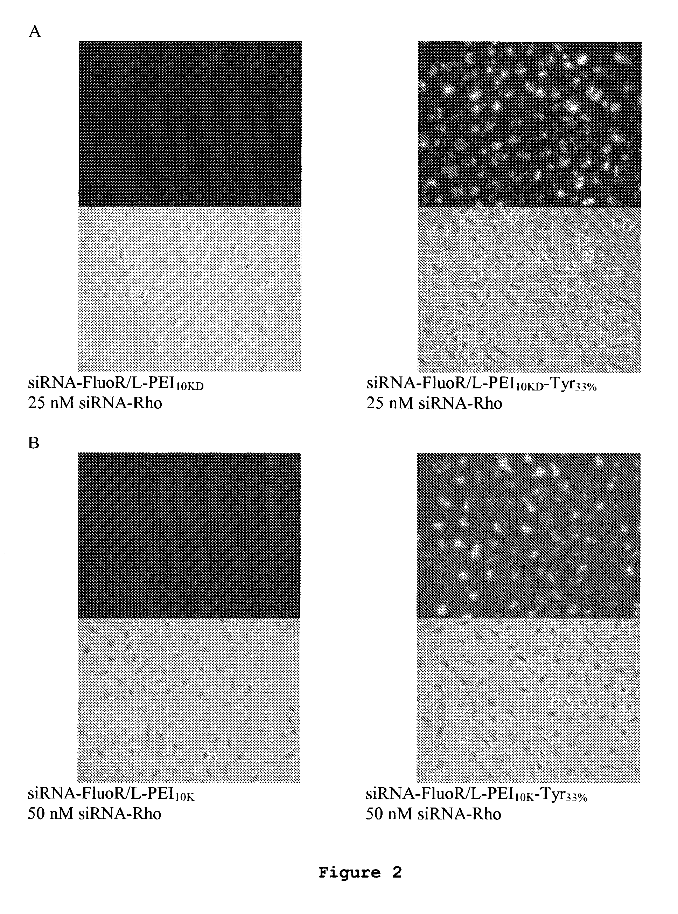

Comparative siRNA delivery potency into cell in culture with linear polyethylenmine and tyrosine modified linear polyethylenimine (L-PEI_{10K}Tyr_{33%}) was investigated using fluorescent siRNA (rhodamine-labelled siRNA, siRNA-FluoR). Small amounts of siRNA-FluoR (final concentration 25 and 50 nM) were complexed with 2 µl of L-PEI_{10K} or L-PEI_{10K}Tyr_{33%} (both stock solutions at 7.5 mM nitrogen) in 50 µl of 50 mM phosphate buffer, pH 6.0. The resulting transfection was added onto A549 cells cultured in complete culture medium containing 10% FBS. Cells were incubated in 24-well tissue culture plate format, for 24 hours before their observation by fluorescence microscopy. The results are given on Figure 2 incubation in 1 ml of complete cell culture medium containing 10% FBS and with 25 (A) or 50 nM (B) siRNA-Rhodamine (Rho) complexed with 2 µl of L-PEI10K or L-PEI_{10K}-Tyr_{33%} (7.5 mM nitrogen) in 50 µl of 50 mM phophate buffer pH 6.0. Before observation, cells were washed with 1ml of PBS-BSA1% and then observed by fluorescence microscopy (ECLIPSE TE2000-S, Nikon), magnification X 200.

Rare punctuate fluorescence within the cytoplasm of cells was observed with L-PEI_{10K}-mediated delivery for the both siRNA concentrations tested. Intense and punctuate fluorescence within all areas of the cytoplasm was observed after siRNA delivery with L-PEI_{10K}Tyr_{33%}. These results show that cellular uptake of siRNA is very efficient using L-PEI₁₀KTyr_{33%} comparatively to the unmodified polyamine.

As a target model for testing the efficiency of the polymers of the invention to mediate the silencing of endogenous reporter gene, we used the A549 cells stably expressing the GL3 luciferase (Photinus pyralis luciferase under the control of SV40 elements).Cells were transfected in 0.55 ml of complete culture medium containing 10% FBS(in 24-well tissue culture plate format) with GL3Luc siRNA, at 20 nM, complexed with 2 µl of 1-PEI_{10K}-Tyr_{x%} (7.5 mM nitrogen) in 50 µl of 50 mM phosphate buffer, pH 6.0. Luciferase gene expression was measured after 48 h incubation period. Experiments were made in triplicates and the luciferase activity was expressed as Relative Light Unit (RLU) normalized by the content of protein in the cell lysates (mg of protein). Then, the silencing efficiency was calculated from the non-transfected cells.

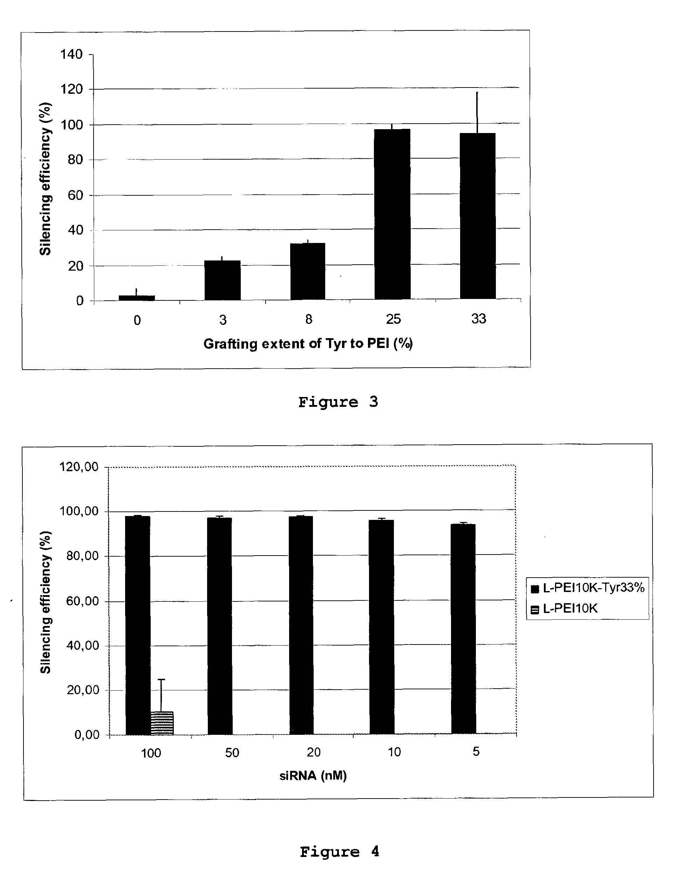

A well defined (validated by Elbashir et al., 2001) and conventional siRNA (siRNAGL3Luc), chemically produced, and sequence-specific GL3Luc siRNA composed of a short dsRNA of 19 nucleotides matching the GL3Luc mRNA and comprising 3'-overhangs of 2 deoxyribonucleotides (dT) was used for the transfection experiments. SiRNA was complexed with unmodified L-PEI_{10K} or modified with tyrosine residue at different extents (3, 8, 25, or 33% of nitrogen modification per polymer) in 50 mM phosphate buffer, pH 6.0. The resulting solution of transfection complexes was added on the cells growing in medium containing serum and cells were finally exposed to siRNA concentration of 20 nM. The results are given on Figure 3. The silencing efficiency was determined 48h post-transfection by measuring the luciferase activity with a standard luminescence assay normalized by the protein content of cell lysates. The luciferase activity (expressed as RLU/mg of protein) was not significantly inhibited (<2%) when the transfection was performed with the unmodified polyamine. When polyamine-tyrosine conjugates were used, the silencing efficiency increased as a function of the grafting extent of tyrosine to polyamine to reach a plateau for 25-33% of modification with 90-95% inhibition of luciferase activity.

### Comparative silencing efficiency of luciferase gene (pGL3) stably expressed by A549-GL3Luc cells by GL3Luc siRNA transfected with the L-PEI_{10K} or L-PEI_{10K}Tyr_{33%} conjugate.

Selective silencing after polymer-mediated siRNA delivery was assessed with A549 cells stably expressing the GL3 luciferase (Photinus pyralis luciferase under the control of SV40 elements). The cells were transfected in 0.55 ml of complete culture medium containing 10% FBS(in 24-well tissue culture plate format) with GL3Luc siRNA, 1 to 100 nM, complexed with 2 µl of 1-PEI_{10K} or 1-PEI_{10K}-Tyr₃₃, conjugate (7.5 mM nitrogen) in 50 µl of 50 mM phosphate buffer, pH 6.0. Luciferase gene expression was measured after 48 h incubation period. Experiments were made in triplicates and the luciferase activity was expressed as Relative Light Unit (RLU) normalized by the content of protein in the cell lysates (mg of protein). Then, the silencing efficiency was calculated from the non-transfected cells.

Specific siRNAGL3Luc was complexed with L-PEI_{10K}Tyr_{33%} in 50 mM phosphate buffer, pH 6.0. Cells were transfected with 5 to 100 nM siRNA. The luciferase activity, determined 48 hours post-transfection, was inhibited up to 98% when the transfection was performed with 5 to 100 nM siRNA. The results are given on Figure 4. As control polymer, unmodified L-PEI₁₀ₖ was shown to inhibit in the same conditions the luciferase activity by 10% at 100 nM. However, luciferase acitivity was not inhibited using lower siRNA concentration from 5 to 50 nM when the transfection was performed with this L-PEI₁₀ₖ.

### Specific gene silencing using siRNA/L-PEI₁₀ₖ-Tyr complex.

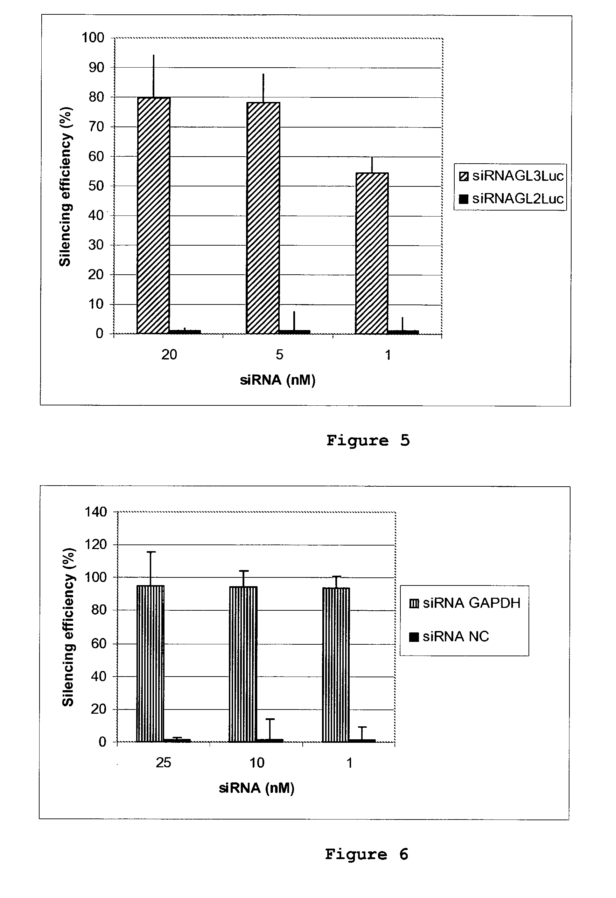

Selectivity of luciferase silencing was tested with the non specific siRNA targeting the GL2Luc gene (siRNA validated by Elbashir et al. 2001) in the same conditions of transfection with L-PEI_{10K}Tyr_{33%}. A549-GL3Luc cells, stably expressing the luciferase gene, were transfected in 0.55 ml of complete culture medium containing 10% FBS(in 24-well tissue culture plate format) with GL3Luc siRNA, 1 to 20 nM, complexed with 2 µl of 1-PEI₁₀K-Tyr_{33%} (7.5 mM nirogen) in 50 µl of 50 mM phosphate buffer, pH 8.0. Luciferase gene expression was measured after 48 h incubation period. Experiments were made in triplicates and the luciferase activity was expressed as Relative Light Unit (RLU) normalized by the content of protein in the cell lysates (mg of protein). Then, the silencing efficiency was calculated from the non-transfected cells. The results are given on figure 5.

The absence of effect on the luciferase activity when cells were transfected with this unrelated sequence, the GL2Luc siRNA, in the same conditions, confirmed a sequence-specific RNA interference.

### Efficient endogenous gene silencing transfection of siRNA complexed with 1-PEI_{10K}-Tyr_{33%}.

HeLa cells were transfected with GAPDH siRNA (1 to 25 nM) complexed with 2 µl of 1-PEI_{10K}-Tyr_{33%} (7.5 mM nirogen) in 50 µl of 50 mM phosphate buffer, pH 8.0. GAPDH mRNA level was measured by branched DNA assay after 48 h incubation period and was inhibited by more than 90% using siRNA concentration from 1 to 25 nM. The results are given on Figure 6. As unspecific control, siRNA matching an unrelated sequence (lamin A/C) was transfected in the same conditions. Unspecific control showed no inhibition effect on the GAPDH mRNA level.

### Cationic polymers modified by hydrophobic alpha amino acids or derivatives mediate efficient gene silencing.

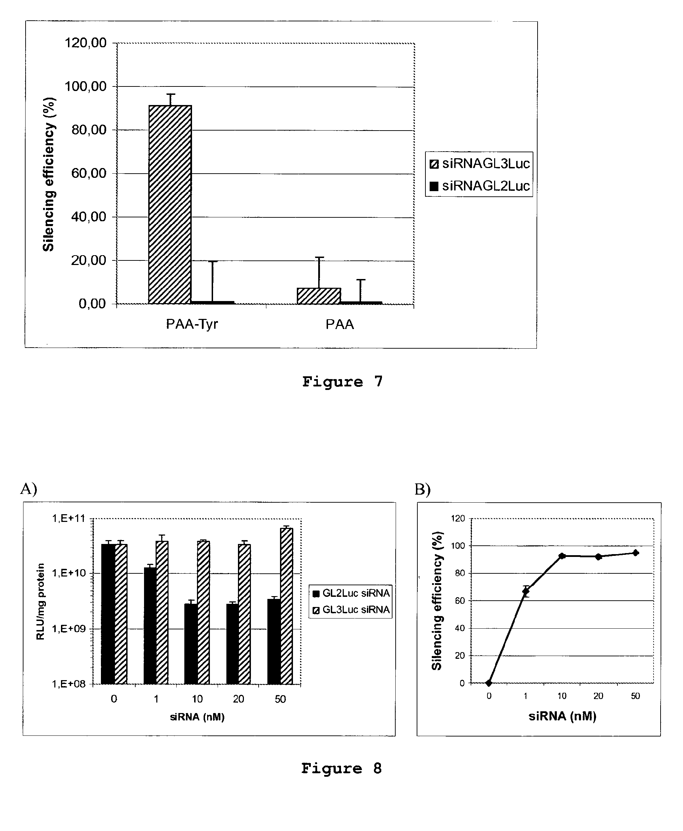

The gene silencing improvement following siRNA delivery into cells in culture with polymer modified with tyrosine residues was also exemplified using the polyallylamine (PAA) having a MW of 17 kDa. PAA was grafted with tyrosine residues with modification extent of nitrogen of 40%. Transfection complexes were prepared with siRNAGL3Luc and 1 µl of PAA_{17K}-Tyr_{40%} in 50 µl of 50 mM phosphate buffer, pH 6.0. A549-GL3Luc cells were transfected in 0.55 ml of complete culture medium containing 10% FBS and with GAPDH siRNA (1 to 25 nM) complexed with 2 µl of 1-PEI_{10K}-Tyr_{33%} (7.5 mM nirogen) in 50 µl of 50 mM phosphate buffer, pH 8.0. GAPDH mRNA level was measured by branched DNA assay after 48 h incubation period. As unspecific control, siRNA matching an unrelated sequence (lamin A/C) was transfected in the same conditions. Experiments were made in triplicates and the GAPDH silencing efficiency was calculated from the endogenously GAPDH level of non-transfected cells. The results are given on Figure 7. PAA_{17K}-Tyr_{40%} provided a silencing of 90% whereas unmodified PAA showed a low and not significant silencing around 10%. In addition, the silencing obtained with PAA_{17K}-Tyr_{40%} was confirmed to be selective because siRNAGL2Luc totally failed to silence the luciferase gene.

Many polymers, including linear or branched polyethylenimine, polyallylamine, or poly-L-Lysine were chemically modified with different hydrophobic alpha amino acids or derivatives such as tyrosine, tryptophane or 3,4-dihydroxy-L-phenylalanine (DOPA) as phenylalanine derivative. Silencing efficiency of these polymer conjugates was tested after transfection of A549 cells stably expressing the GL3 luciferase, using 5 or 20 nM siRNA The results are given in Table 1 hereinafter.

**Table 1: Silencing efficiency of conjugate samples of polyamines modified by aromatic α-amino acid residues with different molecular weight and modification extents (L-PEI: linear polyethylenimine, PAA: polyallylamine, PLK, Poly-L-Lysine).**

| **Polymers** | **MW kDa (polyamine)** | **Grafting (%)** | **MW kDa (conjugate)** | **Silencing (%) at 20 nM siRNA** | **Silencing (%) at 5 nM siRNA** |
|---|---|---|---|---|---|
| **L-PEI-Tyr** | 10 | 25 | 31 | 84+/-18 | 86+/-1 |
| **B-PEI-Tyr** | 25 | 25 | 75,6 | 96+/-1 | 92+/-1 |
| **PAA-Tyr** | 17 | 40 | 47,4 | 91+/-5 | nd |
| **PLK-Tyr** | 22 | 50 | 35 | 87+/-11 | 86+/-8 |
| **L-PEI-Trp** | 10 | 33 | 32,5 | 40+/-9 | nd |
| **PAA-Trp** | 17 | 37 | 49,8 | 87+/-10 | nd |
| **L-PEI-DOPA** | 10 | 23 | 27,9 | nd | 80+/-24 |

| | | | | | |
|---|---|---|---|---|---|
| nd: not determined | | | | | |

The molecular weight of each conjugate is calculated from the mean molecular weight of polyamine and from the percentage of modifications by aromatic α-amino acid residues. Silencing efficiency was determined using A549-GL3Luc cells, stably expressing the luciferase gene. Cells were transfected in 0.55 ml of complete culture medium containing 10% FBS (in 24-well tissue culture plate format) with GL3Luc siRNA, at 5 or 20 nM, complexed with 2 µl of conjugate in 50 µl of 50 mM phosphate buffer, pH 6.0. Luciferase gene expression was measured after 48 h incubation period. Experiments were made in triplicates and the luciferase activity was expressed as Relative Light Unit (RLU) normalized by the content of protein in the cell lysates (mg of protein). Then, the silencing efficiency was calculated from the non-transfected cells.

All the polymer conjugates tested showed high silencing (80% and above) of luciferase gene using low concentrations of siRNA.

High modification extent is required to obtain high gene silencing efficiency. This requirement was also confirmed using the same backbone of polymer but having different MW. The results are given in Table 2 hereinafter

**Table 2: Silencing efficiency of conjugate samples of linear polyethylenimine of different molecular weight modified by 25% with tyrosine residues.**

| **MW kDa (polyamine)** | **MW kDa (conjugate)** | **Silencing (%) at 5 nM siRNA** |
|---|---|---|
| 2 | 6,04 | 67 |
| 10 | 30,2 | 96 |
| 22 | 66,5 | 95 |

The molecular weight of each conjugate is calculated from the mean molecular weight of polyamine and from the percentage of modifications by tyrosine residues. Silencing efficiency was determined using A549-GL3Luc cells, stably expressing the luciferase gene. Cells were transfected in 0.55 ml of complete culture medium containing 10% FBS(in 24-well tissue culture plate format) with GL3Luc siRNA, at 5 nM, complexed with 2 µl of conjugate in 50 µl of 50 mM phosphate buffer, pH 6.0. Luciferase gene expression was measured after 48 h incubation period. Experiments were made in triplicates and the luciferase activity was expressed as Relative Light Unit (RLU) normalized by the content of protein in the cell lysates (mg of protein). Then, the silencing efficiency was calculated from the non-transfected cells.

As shown by said results linear polyethylenimine of 2, 10 or 22 kDa were modified with the same extent of modification with tyrosine residues (25%). All these polymers were able to silence the luciferase gene after transfection of A549-GL3Luc cells with a low siRNA concentration (5 nM).

When analyzed in more details, the high content of hydrophobic alpha amino acids grafted to polyamine strongly increases the mass (MW) of the polymer conjugate (Tables 1 and 2). Particularly, when the initial mass of the polyamine increases 2-3 fold after coupling of hydrophobic alpha amino acids or derivatives, the modified polymer was shown to be efficient for RNA interference. These modified polymers are rather more hydrophobic than cationic. This analysis confirms that hydrophobic interaction drives the overall behavior of polyamine in presence of siRNA and promotes high gene silencing efficiency.

### Efficient gene silencing after co-transfection of plasmid and siRNA complexed with 1-PEI_{10K}-Tyr.

HeLa cells (50 000 cells/well) were co-transfected in 0.55 ml of complete culture medium containing 10% FBS (in 24-well tissue culture plate format) with pCMVLuc plasmid (100 ng, GL2Luc sequence) and either specific GL2Luc siRNA or mismatch GL3Luc siRNA (0 to 50 nM), complexed with 2 µl of PEI_{10K}-Tyr_{19%} in 50 µl of 50 mM phosphate buffer, pH 7.0. Luciferase gene expression was measured after 24 h incubation period. The results are givenon Figure 8. Experiments were made in triplicates and the luciferase activity (A) was expressed as Relative Light Unit (RLU) normalized by the content of protein in the cell lysates (mg of protein). Then, the silencing efficiency (B) was calculated from the ratio of luciferase activities from GL2Luc siRNA- and GL3Luc siRNA-transfected cells.

Specific GL2Luc siRNA inhibited the GL2 Luciferase expression by more than 90% using siRNA concentration from 10 to 50 nM **(****Figure 8****)**. As unspecific control, siRNA matching an unrelated sequence (GL3Luc sequence) was co-transfected in the same conditions. Unspecific control showed no inhibition effect on the luciferase activity. This experiment confirms that **1-PEI_{10K}-Tyr** conjugates are able to simultaneously co-deliver a plasmid encoding a transgene and a specific and active siRNA.

### References

Brummelkamp, T.R., R. Bernards, and R. Agami. 2002. A system for stable expression of short interfering RNAs in mammalian cells. Science. 296:550-3.
Elbashir, S.M., J. Harborth, W. Lendeckel, A. Yalcin, K. Weber, and T. Tuschl. 2001. Duplexes of 21-nucleotide RNAs mediate RNA interference in cultured mammalian cells. Nature. 411:494-8.
Fire, A., S. Xu, M.K. Montgomery, S.A. Kostas, S.E. Driver, and C.C. Mello. 1998. Potent and specific genetic interference by double-stranded RNA in Caenorhabditis elegans. Nature. 391:806-11.
Miyagishi, M., and K. Taira. 2002. U6 promoter-driven siRNAs with four uridine 3' overhangs efficiently suppress targeted gene expression in mammalian cells. Nat Biotechnol. 20:497-500.
Silva, J.M., M.Z. Li, K. Chang, W. Ge, M.C. Golding, R.J. Rickles, D. Siolas, G. Hu, P.J. Paddison, M.R. Schlabach, N. Sheth, J. Bradshaw, J. Burchard, A. Kulkarni, G. Cavet, R. Sachidanandam, W.R. McCombie, M.A. Cleary, S.J. Elledge, and G.J. Hannon. 2005. Second-generation shRNA libraries covering the mouse and human genomes. Nat Genet. 37:1281-8.
Sui, G., C. Soohoo, B. Affar el, F. Gay, Y. Shi, W.C. Forrester, and Y. Shi. 2002. A DNA vector-based RNAi technology to suppress gene expression in mammalian cells. Proc Natl Acad Sci U S A. 99:5515-20.
Verma, S., and F. Eckstein. 1998. Modified oligonucleotides: synthesis and strategy for users. Annu Rev Biochem. 67:99-134. Yu, J.Y., S.L. DeRuiter, and D.L. Turner. 2002. RNA interference by expression of short-interfering RNAs and hairpin RNAs in mammalian cells. Proc Natl Acad Sci U S A. 99:6047-52.

## Claims

1. A composition useful as transfection agent, comprising (i) a polyamine modified by an aromatic amino acid and (ii) a small double-stranded or single-stranded RNA molecule active for RNA interference, wherein the polyamine is a branched or linear polyethylenimine.

2. The composition according to claim 1, wherein the polyamine is a linear polyethylenimine of 2 KD to 220 KD.

3. The composition according to claim 1 or 2, wherein the aromatic amino acids used to modify the polyamines are selected in the group comprising tyrosine, tryptophan and phenylalanine or the derivatives thereof.

4. The composition according to anyone of claims 1 to 3, wherein the RNA is normal or modified, the modification groups being for example 2'-Fluo, 2'-Methoxy, phosphorothioate, LNA or morpholino.

5. The composition according to anyone of claims 1 to 4, wherein the RNA is double stranded or single stranded antisense siRNA or mixtures of single stranded sense antisense siRNA.

6. The composition according to claim 4 or 5, wherein the siRNA molecules are 15-30 mers.

7. The composition according to anyone of claims 1 to 6, comprising polyamines modified by aromatic amino acids such as above defined and double stranded or single stranded siRNA in an isotonic medium, for example NaCl, glucose, a buffer, the concentration of siRNA varying from picomolar to micromolar.

8. The composition according to anyone of claims 1 to 7, further comprising one, or several additives such as PEG, PVA, saccharide, polysaccharide, peptide, protein, vitamins.

9. A method for synthesizing the polyamines modified by aromatic amino acids of the composition according to anyone claims 1 to 8, comprising the use of super-ester of aromatic amino acids activated by Dimethoxytriazine-N-methylmorpholium (DMTMM) in the presence of the polyamines.

10. The method according to claim 9, wherein the synthesis of the polyamines is carried out in a basic buffer such as a borate buffer 200 mM, pH= 7.5-9 or in an aqueous medium in the presence of a base or a water/alcohol mixture.

11. The method according to claim 9 or 10, wherein the percentage of modification of polyamines by aromatic amino acids in said composition varies from 0.01% to 100%.

12. A method for *in vitro* or *ex-vivo* transferring siRNA, comprising using a composition according to anyone of claims 1 to 8.

13. The method according to claim 12 wherein said *in vitro* transfer of siRNA is carried out in cell culture media containing adherent cells or cells in suspension.

14. *In vitro* use of a polyamine modified by an aromatic amino acid, for delivery to an eukaryotic cell of an oligonucleotide providing RNA interference in said cell, said polyamine being a branched or linear polyethylenimine.

## Patentansprüche

1. Zusammensetzung zur Verwendung als Transfektionsmittel, umfassend (i) ein durch eine aromatische Aminosäure modifiziertes Polyamin und (ii) ein kleines doppel- oder einzelstrangiges RNA-Molekül, das zur RNA-Interferenz wirksam ist, worin das Polyamin ein verzweigtes oder unverzweigtes Polyethylenimin ist.

2. Zusammensetzung gemäß Anspruch 1, worin das Polyamin ein unverzweigtes Polyethylenimin von 2 bis 220 KD ist.

3. Zusammensetzung gemäß Anspruch 1 oder 2, worin die aromatischen Aminosäuren, welche zur Modifizierung der Polyamine verwendet werden, aus der Gruppe ausgewählt sind, welche Tyrosin, Tryptophan und Phenylalanin oder deren Derivate umfasst.

4. Zusammensetzung gemäß einem jeden der Ansprüche 1 bis 3, worin die RNA normal oder modifiziert ist, wobei die Modifikationsgruppen beispielsweise 2'-Fluo-, 2'-Methoxy-, Phosphorthioat-, LNA- oder Morpholingruppen sind.

5. Zusammensetzung gemäß einem jeden der Ansprüche 1 bis 4, worin die RNA eine doppelstrangige oder einzelstrangige antisense-siRNA oder Mischungen aus einzelstrangiger sense/antisense-siRNA ist/sind.

6. Zusammensetzung gemäß Anspruch 4 oder 5, worin die siRNA-Molküle 15-30mere sind.

7. Zusammensetzung gemäß einem jeden der Ansprüche 1 bis 6, umfassend, wie oben definiert durch aromatische Aminosäuren modifizierte Polyamine und doppelstrangige oder einzelstrangige siRNA in einem isotonischen Medium, beispielsweise NaCl, Glucose, einem Puffer, wobei die Konzentration an siRNA von picomolar bis micromolar variiert.

8. Zusammensetzung gemäß einem jeden der Ansprüche 1 bis 7, welche des Weiteren ein oder mehrere Additive, wie PEG, PVA, Saccharid, Polysaccharid, Peptid, Protein, Vitamine umfasst.

9. Verfahren zum Synthetisieren der durch aromatische Aminosäuren modifizierten Polyamine der Zusammensetzung gemäß einem jeden der Ansprüche 1 bis 8, umfassend die Verwendung eines Superesters aromatischer Aminosäuren, aktiviert durch Dimethoxytriazin-N-methylmorpholin (DMTMM) in Gegenwart der Polyamine.

10. Verfahren gemäß Anspruch 9, worin die Synthese der Polyamine in einem basischen Puffer, wie einem Boratpuffer von 200 mM, pH= 7,5-9 oder in einem wässrigen Medium in Gegenwart einer Base oder in einem Wasser-/Alkoholgemisch durchgeführt wird.

11. Verfahren gemäß Anspruch 9 oder 10, worin der Anteil der Modifikation der Polyamine mit aromatischen Aminosäuren in der Zusammensetzung von 0,01 % bis 100 % variiert.

12. Verfahren zur in-vitro- oder ex-vivo-Übertragung von siRNA, umfassend die Verwendung einer Zusammensetzung gemäß einem jeden der Ansprüche 1 bis 8.

13. Verfahren gemäß Anspruch 12, worin der in-vitro-Transfer von siRNA in einem Zellkulturmedium durchgeführt wird, welches anhaftende Zellen oder Zellen in Suspension enthält.

14. In-vitro-Verwendung eines durch eine aromatische Aminosäure modifizierten Polyamins zur Beförderung eines Oligonukleotids, das eine RNA-Interferenz in der Zelle liefert, in eine eukariotische Zelle, wobei das Polyamin ein verzweigtes oder unverzweigtes Polyethylenimin ist.

## Revendications

1. Composition utile comme agent de transfection, comprenant (i) une polyamine modifiée par un acide aminé aromatique et (ii) une petite molécule d'ARN double brin ou simple brin active pour l'interférence d'ARN, dans laquelle la polyamine est une polyéthylèneimine ramifiée ou linéaire.

2. Composition selon la revendication 1, dans laquelle la polyamine est une polyéthylènimine linéaire de 2 KD à 220 KD.

3. Composition selon la revendication 1 ou 2, dans laquelle les acides aminés aromatiques utilisés pour modifier les polyamines sont choisis dans le groupe comprenant la tyrosine, le tryptophane et la phénylalanine ou les dérivés de ceux-ci.

4. Composition selon l'une quelconque des revendications 1 à 3, dans laquelle l'ARN est normal ou modifié, les groupes de modification étant par exemple 2'-fluo, 2'-méthoxy, phosphorothioate, LNA ou morpholino.

5. Composition selon l'une quelconque des revendications 1 à 4, dans laquelle l'ARN est un siARN antisens double brin ou simple brin ou des mélanges de siARN sens/antisens simple brin.

6. Composition selon la revendication 4 ou 5, dans laquelle les molécules de siRNA sont de 15 à 30 mers.

7. Composition selon l'une quelconque des revendications 1 à 6, comprenant des polyamines modifiées par des acides aminés aromatiques telles que définies ci-dessus et un siRNA double brin ou simple brin dans un milieu isotonique, par exemple du NaCl, du glucose, un tampon, la concentration en siRNA variant de l'ordre du picomolaire au micromolaire.

8. Composition selon l'une quelconque des revendications 1 à 7, comprenant en outre un, ou plusieurs additifs tels que PEG, PVA, saccharide, polysaccharide, peptide, protéine, vitamines.

9. Procédé de synthèse des polyamines modifiées par des acides aminés aromatiques de la composition selon l'une quelconque des revendications 1 à 8, comprenant l'utilisation de super-ester d'acides aminés aromatiques activé par du diméthoxytriazine-N-méthylmorpholium (DMTMM) en présence des polyamines.

10. Procédé selon la revendication 9, dans lequel la synthèse des polyamines est effectuée dans un tampon basique tel qu'un tampon borate 200 mM, pH = 7,5 à 9 ou dans un milieu aqueux en présence d'une base ou d'un mélange d'eau/alcool.

11. Procédé selon la revendication 9 ou 10, dans lequel le pourcentage de modification des polyamines par des acides aminés aromatiques dans ladite composition varie de 0,01 % à 100 %.

12. Procédé de transfert *in vitro* ou *ex vivo* de siARN, comprenant l'utilisation d'une composition selon l'une quelconque des revendications 1 à 8.

13. Procédé selon la revendication 12, dans lequel ledit transfert *in vitro* de siRNA est effectué dans un milieu de culture cellulaire contenant des cellules adhérentes ou des cellules en suspension.

14. Utilisation *in vitro* d'une polyamine modifiée par un acide aminé aromatique, pour la délivrance à une cellule eucaryote d'un oligonucléotide procurant une interférence d'ARN dans ladite cellule, ladite polyamine étant une polyéthylèneimine ramifiée ou linéaire.
